# EUROPEAN PATENT APPLICATION

(11) **EP 4 589 601 A1**
(43) Date of publication of application: **23.07.2025**
(21) Application number: 24152437.0
(22) Date of filing: 17.01.2024
(51) Int. Cl.: G16H 40/40, G16H 40/60, A61B 5/00, A61B 17/00, A61B 90/00, G01R 31/28, G16H 10/60

(54) **METHOD, DEVICE AND SYSTEM FOR MANAGING A PLURALITY OF MEDICAL DEVICES**

(71) Applicant: Koninklijke Philips N.V., 5656 AG Eindhoven (NL)
(72) Inventor: LEUSSLER, Christoph Günther, 5656 AG Eindhoven (NL); VOGTMEIER, Gereon, 5656 AG Eindhoven (NL); WEIß, Steffen, 5656 AG Eindhoven (NL); JOHNSON, Mark Thomas, 5656 AG Eindhoven (NL)
(74) Representative: Philips Intellectual Property & Standards

(57) **Abstract**

The present invention relates to device, system and method of managing a plurality of medical devices configured for use during a medical procedure of a subject. The method comprises the steps of obtaining test data from a medical device of the plurality of medical devices acquired through one or more device tests of the medical device while not in use for a medical procedure of a subject; processing the obtained test data to determine if the medical device is safe and/or fully functioning and/or ready to use for a medical procedure; and storing and/or providing medical device data indicating the result of the determination of the medical device.

## Description

### FIELD OF THE INVENTION

The present invention relates to method, device and system for managing a plurality of medical devices configured for use during a medical procedure of a subject, such as a patient or other person. The present invention relates further to a test module for use in such a system.

### BACKGROUND OF THE INVENTION

Increasing demands on care systems put additional pressure on already stretched radiology departments. The need to do more with less, reductions in reimbursements and increasing case complexity continue to bring challenges with staffing, skill variability, and quality standardization into sharp focus. The Philips Radiology Operations Command Center (ROCC) is a multi-vendor, multi-modality, multi-site virtualized imaging solution that seamlessly connects imaging experts at a command center with technologists at scan locations across their organization. Technologists can give experts secure access to the console and receive help with completing a procedure or adjusting an imaging protocol. Experts can connect with technologists and view scanner console screens concurrently to provide virtual assistance or cross-training. Moreover, the technologist needs to assess and access individual medical devices (also called clinical device, such as coils and vital signs monitoring) and selection of those. As a further clinical service, the ROCC may select and decide about the required and needed components for the planned MRI scans and consider their impact on image quality and total cost of ownership.

Large hospitals do have many MRI scanners organized and controlled by investment companies. To save money, clinical hardware like RF coils and vital sensing as well as therapy devices are shared between the systems. The medical devices need to be continuously serviced with respect to patient safety and disinfection. Typically, each scanner is equipped with about 10 dedicated coils, which are often not used simultaneously. Today these devices are stored directly at the scanners. They cannot be monitored, located, checked, and controlled remotely and are not shared between different clinical diagnostic scanners.

### SUMMARY OF THE INVENTION

It is an object of the present invention to manage a plurality of medical devices configured for use during a medical procedure of a subject.

In a first aspect of the present invention a method of managing a plurality of medical devices configured for use during a medical procedure of a subject is presented, the method comprising:
- obtaining test data from a medical device of the plurality of medical devices acquired through one or more device tests of the medical device while not in use for a medical procedure of a subject;
- processing the obtained test data to determine if the medical device is safe and/or fully functioning and/or ready to use for a medical procedure; and
- storing and/or providing medical device data indicating the result of the determination of the medical device.

In a further aspect of the present invention a corresponding device is presented.

In a still further aspect of the present invention a system is presented comprising:
- a test module configured to hold and/or store the plurality of medical devices and to carry out one or more device tests with a medical device while not in use for a medical procedure of a subject to generate test data; and
- a device as disclosed herein configured based on the generated test data for managing the plurality of medical devices.

According to another aspect of the present invention, a test module for use in a system as disclosed herein is presented, the test module being configured to
- hold and/or store a plurality of medical devices; and
- carry out one or more device tests with a medical device while not in use for a medical procedure of a subject to generate test data.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, test module, computer program and medium have similar and/or identical preferred embodiments as the claimed method, in particular as defined in the dependent claims and as disclosed herein.

The present invention is based on the idea to check medical devices before they are actually used in a medical procedure, such as an imaging scan or a therapeutic procedure. The check may be done with respect to one or more aspects in a test device that may be configured to hold and/or store multiple medical devices and to perform one or more tests of a medical device while it is not in use. This ensures that a pre-check is made, preferably for every medical device after an actual use and before a subsequent use, so that the medical device is ready to use for the subsequent use. Through the check medical device data are obtained which are provided and/or stored so that the condition and/or parameters of the respective medical device are known and can thus be taken into account in the subsequent use of this medical device.

In addition, it can thus be ensured that the medical device is safe and/or fully functioning and/or ready to use at the point in time when needed for a particular medical procedure. For this purpose, hardware and/or software checks of the medical devices are performed. For instance, hardware and/or software compatibility with respect to SNR and signal integrity may be checked, with highest priority on quality and safety, in particular for clinical imaging and further clinical applications such as therapy and interventional applications. In this way, patient safety, quality, and integrity (with respect to medical devices, workflow, storage, remote operator, etc.) can be improved.

According to a preferred embodiment, the method further comprises a step of controlling a test module to carry out the one or more device tests, the test module being configured to hold and/or store the plurality of medical devices and to carry out one or more device tests. Today, in a typical MR suite the standard set of coils is stored and located in a kind of cupboard somewhere in the MRI room. During the day individual patients having different size and constitution are scanned with different anatomical scans. Dedicated coils and devices must hence be ordered. The test module controlled according to this embodiment may be a mobile service module where clinical staff collects and prepares required medical devices for the medical procedure, e.g., special diagnostic and therapy sessions.

The test module may directly connect to a diagnostic system via an interface and share dedicated medical devices required for the medical procedure. The interface can be wireless or galvanic or an optical link. Further, in the test module the one or more device tests can be carried out and test data can be acquired which are then used to check if the medical device is safe and/or fully functioning and/or ready to use for a medical procedure for which the medical device is configured. Through the use of the test module, transportation of medical devices between apparatus used for the medical procedure, such as a diagnostic scanner, and remote storage may thus be avoided.

According to a further embodiment, the test module is controlled to carry out one or more of the following device tests:
- check of detuning functionality, in particular amplitude, phase and/or latency;
- check for open circuits;
- check for sensitivity, in particular expected SNR, and/or spurious signals;
- check for correct functioning of one or more integrated sensors;
- check for calibration, in particular for time alignment, gain and/or further tuning;
- check of quality and/or signal integrity, in particular with respect to filter settings, clock frequency, tuning and/or time delay;
- check for compatibility;
- check for temperature distribution and/or hot spots of the medical device; and
- check for electromechanical stability.
Each of these checks is relevant for one or more of the aspects of safety, functionality and usability. In addition, patient comfort may be an issue (e.g., hot spots at a medical device exceeding a threshold may not be acceptable for patient comfort and safety).

According to a further embodiment, the test module is controlled to carry out one or more of:
- disinfection procedure for disinfecting the medical device;
- configuring one or more medical devices according to a desired configuration;
- switching on or off one or more medical devices; and
- charging of a battery and/or capacitor of a medical device.

These actions ensure that the medical device is ready to use for a next use during a medical procedure and thus avoids any time delay for any such actions when a medical device shall be used.

Preferably, the method further comprises the step of performing the steps of obtaining and processing test data and of storing and/or providing medical device data for the plurality of medical devices arranged in a test module, in particular for each medical device once it has arrived in the test module. Thus, the preferred handling is that each medical device is tested/check directly after its use when it is put into the test module so that it can be used again as soon as possible and is ready to use for a subsequent medical procedure. Manual handling for performing such actions with the medical devices can thus drastically reduced or completely avoided.

According to an embodiment medical device data are stored and/or provided including one or more of
- information if the medical devices and/or which part of the medical device is safe and/or fully functioning and/or ready to use for a medical procedure;
- signal quality and/or signal integrity electromechanical stability of the medical device;
- calibration information of the medical device; and
- charging state of the medical device.

These medical device data can be used for selecting which medical device to use for a desired medical procedure and/or to evaluate and/or correct any measurements taken by the respective medical device and/or for controlling the respective medical device, which will further improve the quality and accuracy of the medical procedure. The status of the charging state of a medical device is important for patient and device safety. The medical device needs sufficient battery power to provide device functionality during an imaging or other clinical procedure.

According to an embodiment, the method further comprises the step of determining which and/or how one or more device tests shall be carried out based on one or more of
- medical device data generated earlier,
- device information characterizing the medical device,
- procedural information indicating one or more medical procedures for which the medical device can be used, and
- use information including information regarding the next scheduled medical procedure and/or the next subject.

This further improves the workflow and makes sure a used medical device is checked correctly and sufficiently.

Generally, all kinds of medical devices may be used in the context of the present invention. The plurality of medical devices may particularly include medical devices configured for use
- during a medical imaging procedure, in particular during a magnetic resonance imaging procedure, an X-ray imaging procedure, a computed tomography imaging procedure or an ultrasound imaging procedure, and/or
- during a medical treatment procedure, in particular a radiotherapy procedure or hyperthermia application, and/or
- in any diagnostic and/or interventional procedure and/or measurement data acquisition procedure.

Thus, the medical devices may, for instance, include (but are not limited to) one or more of medical resonance coils, vital signs sensors, motion sensor, active markers, transmit and receive coils, local cameras, temperature sensors, measurement data acquisition equipment, and devices for drug delivery and anesthesia.

In another embodiment, the method further comprises the step of selecting one or more medical devices for use in a scheduled medical procedure and/or one or more operations to be carried out in the scheduled medical procedure based on one or more of
- the medical device data,
- device information characterizing the medical device,
- procedural information indicating one or more medical procedures for which the medical device can be used, and
- use information including information regarding the next scheduled medical procedure and/or the next subject and/or persons present in the room of the next scheduled medical procedure.

This further optimizes the workflow and avoids the need for clinical staff to decide which medical device to use for a scheduled medical procedure. Errors by selecting a wrong or defect or otherwise inappropriate medical device can thus be avoided or at least reduced.

The method may further comprise one or more of the steps of
- outputting selection information indicating the selected medical device to a user;
- controlling a user interface to output the selection information, in particular a user interface arranged at a command center, a medical apparatus configured to carry out or be used in a medical procedure, and/or a test module configured to hold the plurality of medical devices and to carry out one or more device tests; and
- controlling a test module to provide the selected medical device, the test module being configured to hold the plurality of medical devices and to carry out one or more device tests.

Such steps further contribute to the desired improvement of the workflow and the avoidance of delays and errors with respect to the use of the medical device.

In an implementation for use in the context of an MRI system, the following specific aspects are preferably checked before a coil is made available for scanning: local specific absorption rate (SAR) to ensure patient safety, signal-to-noise ratio (SNR) and signal integrity to ensure high image quality, and high quality detuning of coil elements to avoid the generation of artifacts in compressed sense imaging due to low quality detuning.

In another embodiment, the obtained test data may be stored and/or provided along with the medical device data. This ensures that they may be used later e.g. for monitoring any changes of the test data that may allow recognizing if a medical device has a reduced reliability or should no longer be used.

The system according to the present invention comprises a test module configured to hold and/or store the plurality of medical devices and to carry out one or more device tests with a medical device while not in use for a medical procedure of a subject to generate test data; and a device as disclosed herein that is configured based on the generated test data for managing the plurality of medical devices. It may further comprise a plurality of medical devices configured for use during a medical procedure of a subject, and/or a medical apparatus configured to carry out or be used in a medical procedure, and/or a test module comprising one or more test units, each test unit comprising one or more sensors configured to carry out one or more of the device tests and to provide the test data, in particular to identify if and/or which medical device is located in the test unit. These sensors may be active and/or passive sensors. For instance, one or more of electromagnetic sensors, a sensor array, and mechanical sensors and actuators for testing mechanical stability may be used.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of a first embodiment of a system according to the present invention;
Fig. 2 shows a schematic diagram of a second embodiment of a system according to the present invention;
Fig. 3 shows a flowchart of a first embodiment of a method according to the present invention;
Fig. 4 schematically shows two embodiments of test units of a test module;
Fig. 5 shows diagrams illustrating various embodiments of test elements that may be used in a test module;
Fig. 6 shows a schematic diagram of a third embodiment of a system according to the present invention;
Fig. 7 shows a schematic diagram of a fourth embodiment of a system according to the present invention;
Fig. 8 shows a schematic diagram of a fifth embodiment of a system according to the present invention; and
Fig. 9 shows a flowchart of a second embodiment of a method according to the present invention.

### DETAILED DESCRIPTION OF EMBODIMENTS

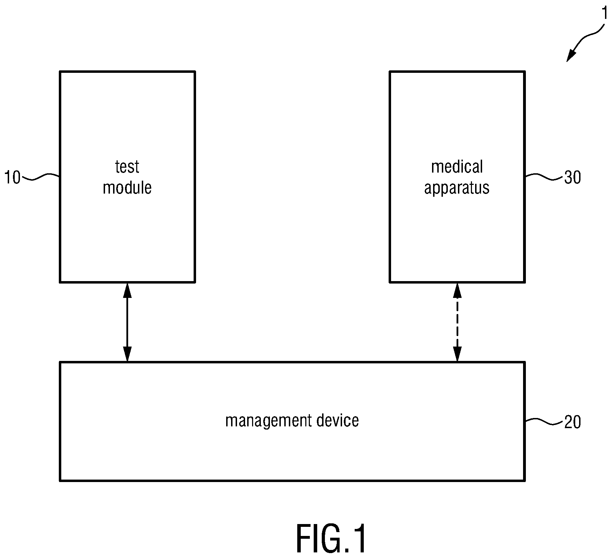

Fig. 1 shows a schematic diagram of a first embodiment of a system 1 according to the present invention for managing a plurality of medical devices for use during a medical procedure of a subject, e.g., a patient or other person. For various medical procedures, such as imaging procedures (e.g., a medical scan) or therapy procedures (e.g., a radiotherapy or surgical procedure) various medical devices (e.g., coils, vital signs sensors, measurement devices, interventional/surgical tools, cameras, and so on) may be used, which should be safe, fully functioning and ready to use at the point in time when needed for the medical procedure. This object is dealt with and solved by the system 1.

The system 1 comprises a test module 10 (also called service module) that is configured to hold and/or store the plurality of medical devices and to carry out one or more device tests with a medical device while not in use for a medical procedure of a subject to generate test data. The system 1 further comprises a device 20 according to the present invention (herein also called management device) for managing the plurality of medical devices based on the test data generated by the test module 10.

Preferably, the test module 10 directly connects to the device 20 via a (wireless or wired) interface and shares test data of the medical devices required for the desired medical procedure with the device 20. Via this connection (or a separate connection) the device 20 may control the test module 10, e.g., to instruct the test module which tests to carry out. The device 20 may also connect with a medical apparatus 30, e.g., a diagnostic imaging scanner, to control the medical apparatus and/or inform the medical apparatus of the medical device that is used in the next medical procedure and/or to obtain information from the medical apparatus 30 regarding the medical procedure (e.g., which type of medical procedure is carried out or how a medical device performed or if there is any defect).

The test module 10 may be implemented as mobile service module where clinical staff collects and prepares required medical devices for special diagnostic and/or therapy sessions. Transportation of medical devices between the medical apparatus 30 used for carrying out the medical procedure and the test module 10 may thus be avoided.

In an exemplary application, the system may be used in the context of MR imaging of patients, where multiple different coils are used for different types of scans of different body parts of different patients. During the day individual patients (having different size, weight, constitution, etc.) are subjected to MRI scans with different anatomical scans. An essential problem tackled by present invention is the lack of a pre-scan check of the different RF coils. One or more (preferably all) of the following specific aspects can be checked by the test module 10 before a coil is made available for scanning: patient safety (local SAR), image quality (SNR and signal integrity) and detuning of coil elements (low quality detuning may generate artifacts in compressed sense imaging).

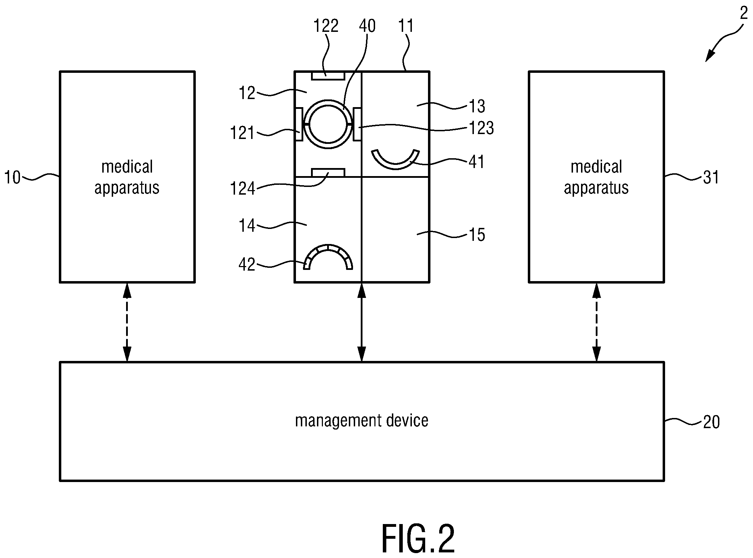

Fig. 2 shows a schematic diagram of a second embodiment of a system 2 according to the present invention. In this embodiment, the system 2 further comprises a plurality of medical devices 40, 41, 42 configured for use during a medical procedure of a subject. The medical devices may, e.g., include one or more of (but not limited to): medical resonance coils (RF coils), vital signs sensors (pulse oximeters, SpO2 sensors, respiration sensors, heart rate sensors, ECG sensors, blood pressure measurement equipment, etc.), motion sensor, active markers, transmit and receive coils, local cameras, temperature sensors, and measurement data acquisition equipment. For instance, in an exemplary use of the system 2 shown in Fig. 2, the medical devices are configured for use in an MRI scan and include a head coil 40, a body coil 41 and a knee coil 42.

The system 2 further comprises a test module 11 comprising one or more test units 12, 13, 14, 15. Each test unit comprises one or more sensors 121, 122, 123, 124 (only shown explicitly for test unit 12) configured to carry out one or more of the device tests and to provide the test data. In the exemplary use of the system 2 for medical devices configured for use in an MRI scan, the coils may be checked by distributed inductive or capacitive electromagnetic sensors located in a fixation or frame of the respective test unit, which locates the medical device. The test units 12-15 may additionally be configured to identify if and/or which medical device is located in the respective test unit. Preferably, the test units 12-15 are configured identically, but they may alternatively be configured differently (e.g., have different sizes and/or different sensors and/or be configured in other ways for certain medical devices and/or certain tests). Electromagnetic tests may be realized by using different forms of signals, like continuous or pulsed signals, signal/noise bursts with different frequency, and amplitude stepping.

In the system 2 the test module 11 is shared among two medical apparatuses 30, 31, e.g. two MRI scanners, so that the number of medical devices can be limited and only a single test module 11 is required for two (or more) medical apparatuses. For instance, coils can be shared between two or more MRI rooms / MR scanners in a way that optimizes the usage of special coils and avoids conflicts as the logistics is linked to the list of patients and related protocols/scans. The shielding between two rooms can thus still be kept intact and just the movement of coils may be possible.

The device 20 may be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor or computer in the room of the operator who operates or monitors the medical procedure may represent the device 20, which may execute a computer program that may be stored in a memory that is accessed by the processor or computer.

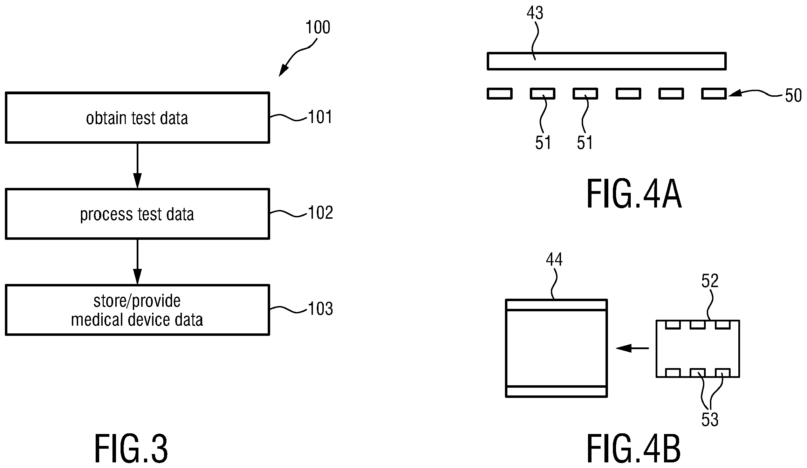

Fig. 3 shows a flowchart of a first embodiment of a method 100 of managing a plurality of medical devices configured for use during a medical procedure of a subject according to the present invention. The steps of the method 100 may be carried out by the device 20, wherein the main steps of the method 100 are carried out by a processing unit of the device 20. The method 100 may e.g. be implemented as computer program running on a computer or processor.

In a first step 101, test data are obtained from a medical device of the plurality of medical devices acquired through one or more device tests of the medical device while not in use for a medical procedure of a subject. For instance, once the medical device (e.g., medical device 40) is placed into a test unit (e.g., test unit 12) of the test module (e.g., test module 11), one or more tests are carried out to check one or more of safety, quality, readiness, etc. of the medical device.

In a second step 102, the obtained test data are processed to determine if the medical device is safe and/or fully functioning and/or ready to use for a medical procedure. For instance, it may be checked if the battery (or capacitor) of the medical device needs to be charged (and it is charged if needed) and the sensitivity of the medical device may be checked to obtain, as part of the test data, sensitivity information which may be taken into account for a subject evaluation of measurement data acquired by the medical device.

In a third step 103, medical device data indicating the result of the determination of the medical device are storing and/or provided, e.g. to a central storage of medical device data of a plurality of medical devices or to a computer that selects medical devices for subsequent use and/or controls medical devices and/or processes measurement information obtained during a medical procedure.

In general, the tests of a medical device may include one or more hardware (HW) checks and/or one or more software (SW) checks of medical devices to make sure that these medical devices are safe, fully functioning, and ready to use at the point in time when needed for a particular medical procedure. One function is to check HW and SW compatibility with respect to SNR and signal integrity, with a priority on quality and safety for medical procedures, e.g. for clinical imaging or further clinical applications such as therapy and interventional applications.

In an embodiment, the test module may be configured as universal modular platform, which is integrated, mobile, and dockable to a medical apparatus such as an MRI scanner. It stores coils and other medical devices in a defined and controlled order for the upcoming scans.

The test module may switch between different modes: a "maintenance mode" (charging, disinfection, test and check) and an "interactive mode" (SW algorithm and a decision control for guidance and connection during patient preparation with the operator). During the interactive mode, the method may predict or select the next medical device to be used and supports to use it without expert know-how. It may select and release medical devices during preparation of the patient and may also retrieve medical devices after the medical procedure. Furthermore, devices that are defect or incompatible can be blocked.

In the exemplary use for MRI, RF coils that are available, intact and suitable for the next scan with respect to the MRI protocol, the patient status, scanner type and B0 magnetic field, may be presented and delivered to staff for use. If they are used by staff, their release may be documented. This highly controlled and automated management of medical devices prevents the use of wrong medical devices by unexperienced staff which is particularly important in a scenario where local staff may be less experienced/trained.

A 3D sensing/interaction unit may be integrated into the system to cooperate safely with the user/operator with respect to location of patient bed, loading and further parameters to achieve patient and operator safety.

The proposed system may be used as a service for MRI scanner with standard coils. A test module in the form of a mobile platform may be configured in this use case with advanced dedicated coils and solutions (e.g., devices for monitoring or intervention).

Optionally, the proposed system may have access to patient history and scanner status, e.g. to a database storing electronic health records of patients, for instance in a hospital network. Based on this additional information, a decision making rule-based concept may be implemented with respect to upcoming patient and imaging/therapy service.

During the process of docking of the test module and release/presentation of medical devices, the medical apparatus may be safely controlled, e.g. by the management device 10, to prevent start of the medical procedure, e.g., of an MR sequence.

The test module optionally has a connector for device functional tests and functionality to test the medical device and to report about the status. This is desired as a quality check before next usage. In case of a detected defect, a service call can be initiated.

The system and the test module make sure that wireless devices (e.g., coils and physiology sensors) stored in the test module are readily charged to the amount as required for a specific next medical procedure and fully functioning. The function of immediate recharging after a medical device is put back to the module has the advantage that medical devices (such as wireless coils) with less battery capacity can be designed, which are more lightweight. It also obviates the current work step of replacing and recharging batteries (e.g. of a wireless ECG signal transducer).

Optionally, the test module has a disinfection function, e.g. to spray disinfection liquids to the surfaces of the medical device. In an interventional MR system the test module may provide interventional devices and helper devices and serve them in a sterile state.

In another embodiment, a guidance functionality (e.g. audio and/or video) may be provided to show the operator/staff how to use the medical device, e.g., how to place a coil on the patient's body, and how to connect it to the system.

According to an embodiment of the present invention the test module (or service module) may be located close to or even integrated into the medical apparatus, e.g., the magnet of an MRI scanner. The test module contains the medical devices and presents and releases the right medical device to the operator during preparation. The system may be digitally connected to a digital health suite platform to adjust to the patient data/history and to the allowed system parameters of the medical device, e.g., of a coil (SAR model, max B1, allowed anatomy, orientation). The system may be driven by an actuator that rotates or is vertically oriented close to the medical apparatus or fixed to patient bed platform. An integrated sense platform (using, e.g., video and/or radar and/or RFID) is used to facilitate a safe procedure. This simplifies the workflow for the medical device selection.

Fig. 4 schematically shows two embodiments of test units of a test module, for instance a portable test module, with integrated quality check for RF coils and mobile sensors. In the test unit 50 shown in Fig. 4A, a coil 43 is checked by sensors 51. Distributed inductive or capacitive sensors 51 are located in a fixation or frame, which locates the device under investigation. Each individual coil can thus be checked for coil sensitivity, which is a measure for the expected SNR. A further test is the function of the detuning functionality (amplitude, phase, latency), which is directly linked with B1 homogeneity and patient safety (local SAR).

The test data (signals) of the sensors may be digitized and sent to the management device or another processing means, e.g. a server in the cloud. The management device or a cloud-based software module may then decide, for instance based on a threshold or window level-based model, about the allowed selection of the medical device under test. The model may depend on local measured signals and on statistical data of all measured medical devices in a database. The parameters of the software module may be stored in a local module, which allows high quality and safe imaging in case of lost internet connection.

The test unit 52 shown in Fig. 4B is configured as a cylindrical test unit equipped with a cylindrical array of sensors 53. Each sensor is 53 located in a defined position relative to the individual RF coil element 44, in this case a head coil into which the test unit 52 may be positioned. The cylindrical test unit 52 may transmit, for instance via WLAN or in any other wireless or wired way, the measured test data to the management device or a cloud service for further processing.

The test units 50, 52 and fixation mechanics are preferably located in the modular test module. In another embodiment, a local stand-alone medical device can be combined with a special quality check and calibration device.

According to the present invention, one or more of a plurality of the following tests/checks can be performed:
- Check for patient safety: It tests (using a sensor approach as explained below) the function of the detuning functionality (amplitude, phase, latency), which is directly linked with B1 homogeneity and patient safety (local SAR).
- Check for open circuits: Using a sensor approach as explained below
- Check for coil sensitivity and spurious signals: Each individual coil is checked (using a sensor approach) for coil sensitivity, which is a measure for the expected SNR
- Check for coil detuning: It tests (using a sensor approach) the function of the detuning functionality (amplitude, phase, latency), which is directly linked with B1 homogeneity and patient safety (local SAR).
- Check for correct functioning of integrated sensors: Mobile digital sensors such as vital signs monitoring devices produce spurious noise. Also RF coils with integrated sensors can downgrade signal integrity. Root causes are often broken or mechanical aging of EMC shielding, software updates and shift of clock frequency. The spurious noise level and signals can be checked.
- Check for calibration: The medical device is measured with defined signal levels and a calibration is realized by the setup. Calibration may be realized for time alignment, gain and further tuning steps (integrated filters...). This test of quality and signal integrity is an active test, which means that during the procedure parameters (filter settings, clock frequency, tuning, time delay, etc.) can interactively be tuned for optimal signal quality and patient safety before the coil/device is located in the clinical diagnostic scanner.
- Check for electromechanical stability: The above sensing can be applied in combination with an actuator to check for mechanical stability and lose contacts of the medical device under test. The actuator may inject mechanical pulsed or periodical waves to the object. In a different setup, the test object is partly bended or distorted at critical parts.

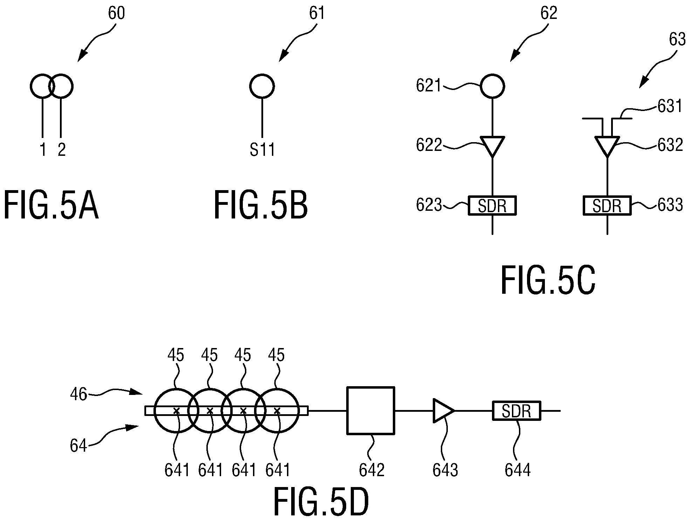

Fig. 5 shows diagrams illustrating various embodiments of test elements that may be used in a test module.

Fig. 5A shows a test element 60 in the form of two overlapping loops for detecting the detuning efficiency using pick up probes formed as loop elements (semi-rigid coax wire). The two fixed overlapping loops are located over the plane surface of an MRI loop coil. The sensor position is not critical. The MRI coil electronics is switched on and off and a clear signal difference is detected via a S21 measurement. An acceptable value of detuning is e.g. a value of S21 = +/-1dB.

Fig. 5B shows a test element 61 in the form of a single loop for an S11 measurement that provides information of the resonant circuit in front of the preamplifier of an MRI coil. This measurement can detect an unconnected/unsoldered conductor or a broken conductor. When the loop is used as a transmitter antenna, then the signal path of the MRI loop can be characterized with respect to signal gain and unbalanced signal transfer function.

Fig. 5C shows a test element 62 including a loop 621 and another test element 63 including an electrical antenna 631. An MRI coil can generate unwanted spurious signal in the form of self-oscillation of the preamplifier or by spurious signals from DC power step up converters or broken shielding of local digital electronics or software updates. Local test probes as loops or electrical antennas or combinations are connected to broadband preamplifiers 622, 632. The signals of the preamplifiers are fed to a broadband SDR receiver 623, 633 and the frequency spectrum is analyzed. Preamplifiers with self-oscillations can become hot and generate local temperature hotspots which is harmful for the patient.

Fig. 5D shows a test element 64 including multiple sensors 641 located at the positions of the individual coils 45 in a coil array 46. The sensors 641 are connected to a diversity switch 642 and an amplifier 643. The test is performed sequentially. Here, only one SDR/analyzer 644 may be used. The analyzer 644 may not be galvanically connected but using a wireless transceiver. The data is analyzed in the cloud or locally. To reduce the number of datapoints and information the sensor signals are not continuously monitored, but only before the coils are applied or when stored. The signal data of the individual sensors can be measured in an RF lab and are available on request. The test module stores RF coils and other medical devices. The test module may be prepared for the need of the clinical imaging procedure. An optimal image results from the right combination of dedicated RF coils and sensors with the MR imaging sequence. It stores and delivers the right coils to the staff and patient.

Another test element (not shown) may be configured to perform a test of electromechanical stability: The above sensing can be applied in combination with an actuator to check for mechanical stability and lose contacts of the device under test. The actuator may inject mechanical pulsed or periodical waves to the object. In a different setup, the test object is partly bended or distorted at critical parts. The actuators and RF channels for sensing including control of power supply of the different functional groups may be controlled by computer program. Different individual test sequences may be selected and applied for the different medical devices under test.

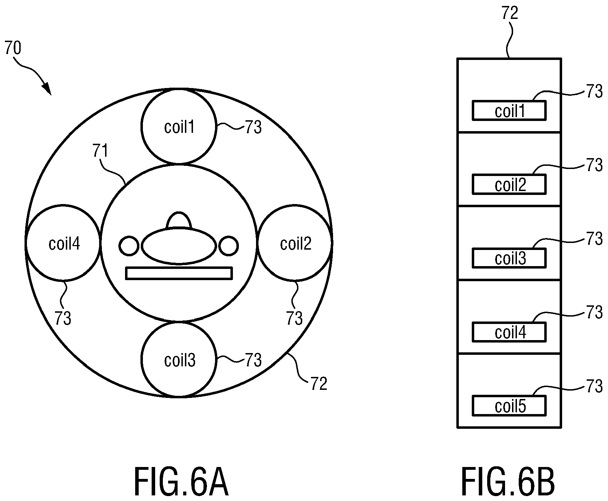

Fig. 6 schematically shows an embodiment of a system 70 in the form of a service module comprising an MRI scanner 71 as an example of a medical apparatus and a test module 72 that is implemented as a revolver like cylindrical rotatable donut on the front (or rear) side of the MRI scanner 71 with integrated coils 73 representing multiple medical devices. Fig. 6A shows a front view and Fig. 6B shows a side view. The system 70 is thus configured as modular service module and is equipped and configured for requirements needed to scan individual patients during a defined time. The configuration of the system for use during an MRI procedure may be planned and controlled by a planning software tool. The system 70 thus enable to present coils and sensors to staff and patients for fast, safe, and optimized workflow.

Optionally, the service module 70 may be part of a Radiology Operations Command Center (ROCC) operated MR suite. In that case, the ROCC has access to the service module to directly and intuitively select optimal coils and to select the most appropriate scan sequence. The status of the service module is monitored at the command center. In some applications, staff interacts with the patient at the back side of the scanner, e.g., for certain interventional applications. In that case, a rotatable service module may be attached at the back of the scanner to provide MR helper devices or interventional devices.

In an alternative embodiment, the test module may be integrated at one or both sides of the medical apparatus, e.g., an MRI scanner. This may provide several advantages: a) more space is provided to store medical devices; b) only the medical device that is at the front side is offered for current use, which avoids ambiguities as with the embodiment of the system 70 shown in Fig. 6, where several coils are in reach; c) the arrangement of medical devices on the side of the medical apparatus avoids the impression that the apparatus, in particular a bore (like an MR bore) for placing the patient becomes longer and darker, which may be an effect of the revolver design shown in Fig. 6. In a variant of this embodiment the space on top of the medical apparatus may be used as well.

This alternative embodiment of the system may thus represent a modular service module as well, which is realized as a circular conveyor system attached at the side of the medical apparatus. The conveyor rotates to present the medical device to be used next to the front position where staff can collect it. Two such circular conveyor systems may be used at both sides of the medical apparatus so that medical device after medical device can be presented from the left and right conveyor system in turns.

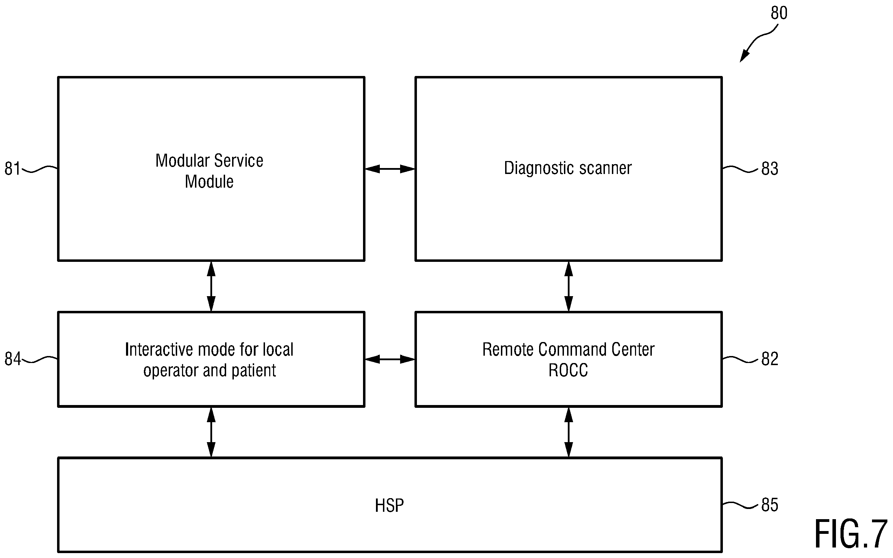

Fig. 7 schematically shows another embodiment of a system 80, in which a modular service module 81 (representing a test module) is connected via a data link to an ROCC 82, a diagnostic scanner 83 (or other medical apparatus), an interactive monitoring unit 84 for local operator and patient, and a healthcare compliant cloud solutions unit (HSP) 85. The modular service module 81 is equipped with sensors (e.g., camera, US, RADAR, RFID). The sensors report the status of configuration and the status of the ongoing preparation of the patient.

The system 80 can switch between different modes:
- maintenance mode: charging, disinfection, test and check;
- interactive mode: SW algorithm and a decision control for guidance and connection during patient preparation with the operator;
- communication mode with ROCC;
- storage mode; and
- mode of mechanical and electrical connection and approach to system.

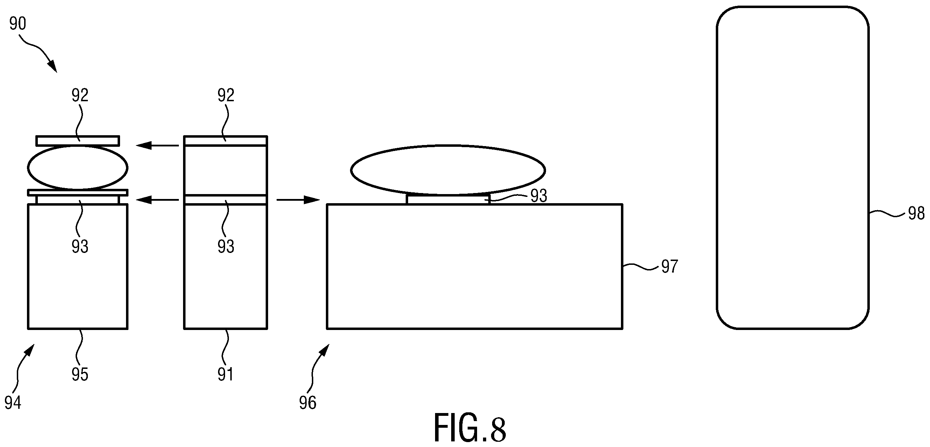

Fig. 8 schematically shows another embodiment of a system 90 in the form of a configurable diagnostic site for mobile X-Ray imaging. It comprises a test module 91 storing, e.g., ECG equipment 92, a device 93 for breathing gating, contrast agent, interventional devices, etc. The test module 91 can be used for a mobile diagnostic device 94 having a mobile patient support 95 and a stationary (or mobile) diagnostic device 96 having a patient support 97. With the advent of low and ultra-low field systems clinical diagnostic and therapy systems can be configured on place. A device 98 according to the present invention, e.g. including a software module, may systematically plan and configure the clinical configuration on place. The test module 91 may be configured to manually or automatically deliver the medical devices by, e.g., a vertical lift.

As explained above, one main application of the present invention is in the field of MRI, where multiple coils are tested and stored for an upcoming scan. The correct body coil may be selected by the device and method as well, i.e., a piece of hardware is selected that is connected to the scanner and has to be tested after this configuration. Other medical devices than the RF receive coils may comprise any components of the MR system or additional devices required to perform the diagnostic scan, which may be checked for electromagnetic compatibility (EMC), signal integrity (SI), and/or power integrity (PI).

Further checks, monitoring and configuration may be made, including a check of the RF and gradient chain (power distribution network), a check of system noise level (footprint of RF noise for corrective maintenance or select/decide parameters for the scan), and a check of compatibility of external devices located close or in the bore (ECG, anesthesia systems, close by X-Ray systems, MR - LINAC, therapy systems, etc.).

For upcoming low-cost ultra-low-field (ULF) MRI (i.e., <0.1 T) for truly point-of-care applications and low field sustainable systems, all these tests should be made as these systems are at the edge of clinical usability. They include using resistive electromagnets that can produce a homogenous field (0.001 T and about 0.05 T). Such ultra-low-field systems have a higher sensitivity to external noise than conventional systems, as there is no fully RF screen enclosure. These systems can be constructed as a mobile device like portable X-Ray systems and can be applied as stand-alone systems for point-of- care solutions or be located fixed in a RF screened room.

For point of care systems these MRI devices can be used in combination with X-Ray or ultrasound systems. A clinical scenario for trauma patients can be X-Ray first and then directly using MRI for further diagnosis or monitoring. MR image degradation is induced by EMC radiation from devices close by.

EMC noise is produced by X-Ray generator, monitors, anesthesia systems, switch mode power supply etc. , sensors using digital electronics. To improve signal integrity the individual devices need to be switched off or being located or oriented in the clinical diagnostic room in a defined position. Not all devices can simply be switched off, as they are connected to cloud networks for data exchange or patient life support etc.

Another field of application is DXR CT, in which medical devices like ECG equipment, contrast pump, and other are used as external but connected medical devices for a fully configured system. Generally, there are no exchangeable detectors or multiple detectors as in MRI, but different types of wireless detectors may be used that are able to connect to a DXR system, in which scenario it has to be ensured that the coupled detector works with the system. In CT the detector normally is not exchanged, but some electrical test of the detector comprising several modules and read-out electronics may be subject to a self-diagnosis by use of the proposed approach.

The modular check procedure for safety, signal noise floor, sensor test, camera test, calibration using a test signal, etc. as described above is generally usable for different medical procedures including different imaging modalities. Generally, all allowed combinations of medical devices described above may be stored and monitored. Allowed configurations may be stored in a database and may be visualized for the user. New medical devices can be checked and integrated in the configuration. Allowed combinations and clinical functions may be checked presented to achieve optimal image quality. The management device and method may further be configured to switch off or shut down processes during the imaging. They may also directly control individual power supplies (e.g., via e- switches) and data connections (WLAN, GSM on/off) or monitors (on/off). Further measures can be realized by defined local additional modular shielding using shielding walls, blankets, etc.

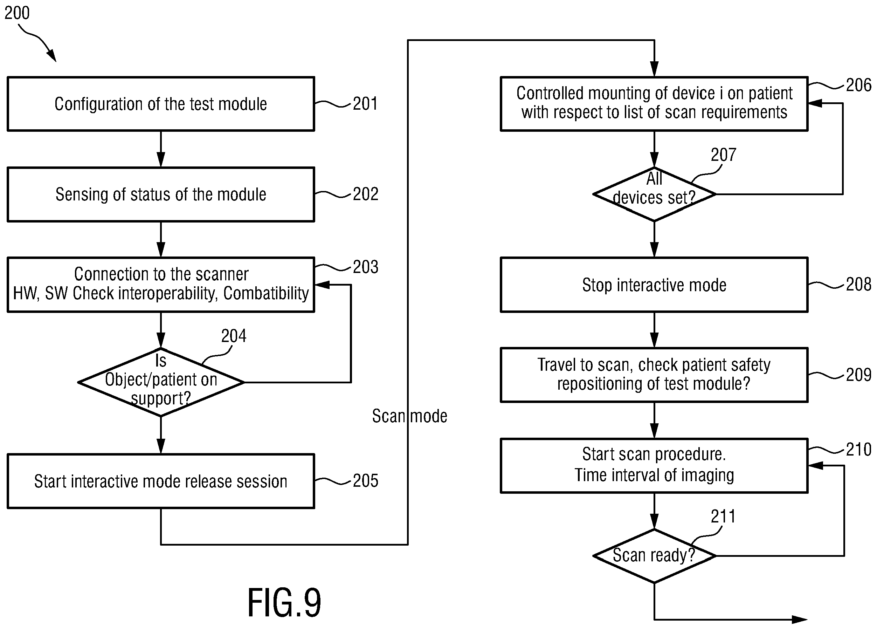

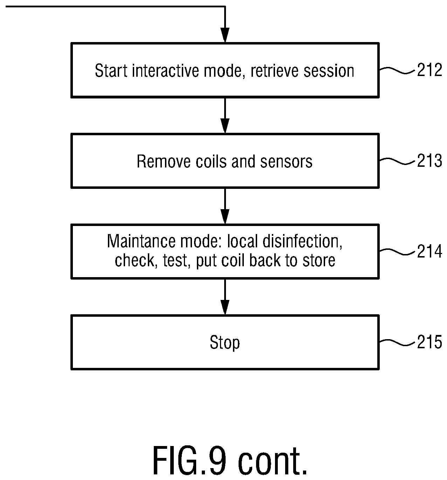

Fig. 9 shows a flowchart of a second embodiment of a method 200 according to the present invention. The method 200 provides an algorithm and decision control for guidance and connection. A software management for verification, control, interaction, connection (verify and approve, validate) is applied. The system switches between interactive mode and scan mode.

In a first step 201 the test module is configured. In step 202 the status of the test module is sensed. In step 203 a connection to the medical apparatus (e.g., a medical scanner) is made. Further, a checks of required medical devices are carried out. In step 204 it is checked if an object or patient is on the patient support (more generally, if a patient is ready for a medical procedure). If not, the method proceeds with step 203; otherwise, in step 205 the interactive mode is started, and a medical procedure session is released.

In step 206 the required medical device(s) is (are) mounted on the patient (or the medical apparatus) with respect to a list of requirements of the medical procedure. In step 207 it is checked if all required medical devices are set. If not, the method proceeds with step 206; otherwise, in step 208 the interactive mode is stopped.

In step 209 the scan mode is started, and it is continued with the medical procedure, for instance the scan. Patient safety is checked, and optionally the test module is repositioned if needed. In step 210 the medical procedure (e.g. a scan) is started for a certain time interval. In step 211 it is checked if the medical procedure is ready. If not, the method proceeds with step 210; otherwise, in step 212 the interactive mode is started again, and the next session is retrieved. In step 213 medical device (e.g., coils and sensors) are removed. In step 214 a maintenance mode is started, in which disinfection, checks, tests and storage of the used medical devices are carried out. In step 215 the method is stopped until the next medical procedure shall be carried out.

In summary, according to the present invention a device, system and test module for managing a plurality of medical devices for use in medical procedures are provided. HW and/or SW checks of medical devices are made to make sure that these medical devices are fully functioning and ready to use at the point in time when needed for a particular medical procedure. The proposed approach may optionally be integrated in a universal modular medical apparatus, such as a coil/device platform (B0 field independent), which may be integrated, mobile, and dockable to a conventional medical apparatus such as an X- Ray or MRI scanner. According to the present invention, medical devices are stored and tested in a defined and controlled order for upcoming medical procedures. Generally, the present invention may be applied for many different medical procedures and apparatuses, in particular all kinds of imaging modalities such as mobile diagnostic imaging, MRI, CT, DXR, PET and others. The proposed method and device may be implemented in software and/or hardware, e.g., as software module implemented locally or remotely.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Method of managing a plurality of medical devices configured for use during a medical procedure of a subject, the method comprising:
- obtaining test data from a medical device of the plurality of medical devices (40-42) acquired through one or more device tests of the medical device while not in use for a medical procedure of a subject;
- processing the obtained test data to determine if the medical device is safe and/or fully functioning and/or ready to use for a medical procedure; and
- storing and/or providing medical device data indicating the result of the determination of the medical device.

2. Method according to claim 1, further comprising:
controlling a test module (10, 11) to carry out the one or more device tests, the test module being configured to hold and/or store the plurality of medical devices and to carry out one or more device tests.

3. Method according to claim 2,
wherein the test module (10, 11) is controlled to carry out one or more of the following device tests:
- check of detuning functionality, in particular amplitude, phase and/or latency;
- check for open circuits;
- check for sensitivity, in particular expected SNR, and/or spurious signals;
- check for correct functioning of one or more integrated sensors;
- check for calibration, in particular for time alignment, gain and/or further tuning;
- check of quality and/or signal integrity, in particular with respect to filter settings, clock frequency, tuning and/or time delay;
- check for compatibility;
- check for temperature distribution and/or hot spots of the medical device; and
- check for electromechanical stability.

4. Method according to claim 2 or 3,
wherein the test module (10, 11) is controlled to carry out one or more of:
- disinfection procedure for disinfecting the medical device;
- configuring one or more medical devices according to a desired configuration;
- switching on or off one or more medical devices; and
- charging of a battery and/or capacitor of a medical device.

5. Method according to claim 2, further comprising:
performing the steps of obtaining and processing test data and of storing and/or providing medical device data for the plurality of medical devices arranged in a test module, in particular for each medical device once it has arrived in the test module.

6. Method according to any one of the preceding claims,
wherein medical device data are stored and/or provided including one or more of:
- information if the medical devices and/or which part of the medical device is safe and/or fully functioning and/or ready to use for a medical procedure;
- signal quality and/or signal integrity electromechanical stability of the medical device;
- calibration information of the medical device; and
- charging state of the medical device.

7. Method according to any one of the preceding claims, further comprising:
determining which and/or how one or more device tests shall be carried out based on one or more of:
- medical device data generated earlier,
- device information characterizing the medical device,
- procedural information indicating one or more medical procedures for which the medical device can be used, and
- use information including information regarding the next scheduled medical procedure and/or the next subject.

8. Method according to any one of the preceding claims,
wherein the plurality of medical devices (40-42) includes medical devices configured for use:
- during a medical imaging procedure, in particular during a magnetic resonance imaging procedure, an X-ray imaging procedure, a computed tomography imaging procedure or an ultrasound imaging procedure, and/or
- during a medical treatment procedure, in particular a radiotherapy procedure or hyperthermia application, and/or
- in any diagnostic and/or interventional procedure and/or measurement data acquisition procedure.

9. Method according to any one of the preceding claims, further comprising:
selecting one or more medical devices (40-42) for use in a scheduled medical procedure and/or one or
more operations to be carried out in the scheduled medical procedure based on one or more of:
- the medical device data,
- device information characterizing the medical device,
- procedural information indicating one or more medical procedures for which the medical device can be used, and
- use information including information regarding the next scheduled medical procedure and/or the next subject and/or persons present in the room of the next scheduled medical procedure.

10. Method according to any one of the preceding claims,
further comprising one or more of:
- outputting selection information indicating the selected medical device to a user;
- controlling a user interface to output the selection information, in particular a user interface arranged at a command center, a medical apparatus configured to carry out or be used in a medical procedure, and/or a test module configured to hold the plurality of medical devices and to carry out one or more device tests; and
- controlling a test module to provide the selected medical device, the test module being configured to hold the plurality of medical devices and to carry out one or more device tests.

11. Computer program comprising program code means for causing a computer to carry out the steps of the method as claimed in any preceding claim when said computer program is carried out on the computer.

12. Device (20) for managing a plurality of medical devices configured for use during a medical procedure of a subject, the device comprising circuitry configured to:
- obtain test data from a medical device of the plurality of medical devices acquired through one or more device tests of the medical device while not in use for a medical procedure of a subject;
- process the obtained test data to determine if the medical device is safe and/or fully functioning and/or ready to use for a medical procedure; and
- store and/or provide medical device data indicating the result of the determination of the medical device.

13. System (1, 2, 70, 80, 90) for managing a plurality of medical devices for use during a medical procedure of a subject, the system comprising:
- a test module (10, 11, 72, 81, 91) configured to hold and/or store the plurality of medical devices and to carry out one or more device tests with a medical device while not in use for a medical procedure of a subject to generate test data; and
- a device (20, 82, 98) according to claim 12 configured based on the generated test data for managing the plurality of medical devices.

14. System according to claim 13,
further comprising one or more of:
- a plurality of medical devices (40-42) configured for use during a medical procedure of a subject, in particular one or more of medical resonance coils, vital signs sensors, motion sensor, active markers, transmit and receive coils, local cameras, temperature sensors, measurement data acquisition equipment, and devices for drug delivery and anesthesia;
- a medical apparatus (30, 71, 83, 94, 94) configured to carry out or be used in a medical procedure; and
- a test module (11) comprising one or more test units (12-15), each test unit comprising one or more sensors configured to carry out one or more of the device tests and to provide the test data, in particular to identify if and/or which medical device is located in the test unit.

15. A test module (10, 11, 72, 81, 91) for use in a system according to claim 13 or 14, the test module being configured to:
- hold and/or store a plurality of medical devices; and
- carry out one or more device tests with a medical device while not in use for a medical procedure of a subject to generate test data.
